# EUROPEAN PATENT APPLICATION

(11) **EP 4 781 940 A1**
(43) Date of publication of application: **29.07.2026**
(21) Application number: 25153789.0
(22) Date of filing: 24.01.2025
(51) Int. Cl.: A61B 18/20

(54) **LIGHT-BASED SKIN TREATMENT DEVICE, COMPUTER-IMPLEMENTED METHOD, AND COMPUTER PROGRAM PRODUCT**

(71) Applicant: Koninklijke Philips N.V., 5656 AE Eindhoven (NL)
(72) Inventor: ZHANG, Jun, Eindhoven (NL); LIN, Xiaoyu, Eindhoven (NL)
(74) Representative: Philips Intellectual Property & Standards

(57) **Abstract**

There is provided a light-based skin treatment device comprising a treatment light source, a sensor light source, a light sensor and a controller. The treatment light source is adapted to provide treatment light to the skin. The sensor light source is adapted to irradiate a skin portion with sensor light. The light sensor is arranged to receive reflected sensor light from the skin portion, and to generate a sensor signal representative of a skin color. The controller is configured to receive from the light sensor a first sensor signal representative of a skin color of a first skin portion. The controller is configured to receive from the light sensor a second sensor signal representative of a skin color of a second skin portion, to determine first monochromatic data based on the first sensor signal, and to determine second monochromatic data based on the second sensor signal. The controller is configured to determine a difference between the first monochromatic data and the second monochromatic data. The controller is configured to generate a treatment signal based on the difference.

## Description

### FIELD OF THE INVENTION

The invention relates to light-based skin treatment device, such as an intense pulsed light (IPL) device. The invention further relates to a computer-implemented method. The invention further relates to a computer program product.

### BACKGROUND OF THE INVENTION

Photo-epilation is well known for hair removal and hair growth reduction. Home-use consumer devices are commercially available, such as the Lumea (TM) of Philips (TM). Home-use devices typically use Intense Pulsed Light technology (IPL) from e.g. a Xenon flash lamp at a relatively low fluence (up to 6.5 J/cm²), as compared to professional devices for permanent photo-epilation, that use fluences in excess of 10 J/cm².

IPL technology uses a high-powered, hand-held, flash lamp to deliver an intense, visible, broad-spectrum pulse of light, generally in the visible spectral range of 400 to 1200 nm. Cutoff filters are for example used to selectively filter out shorter wavelengths, especially potentially damaging ultra violet light. The resulting light has a spectral range that targets specific structures and chromophores, in particular the melanin pigment in hair.

The light absorbed by melanin in the hair and hair matrix generates heat that puts the hair follicles in a sleep state. When the treatment is repeated in intervals of 2 to 4 weeks, a long-lasting hair reduction result is obtained.

To treat a complete body part, such as a whole leg, the device is moved by the user along the body part. The user holds the device at a position at which the device irradiates a portion of the skin with a pulse of treatment light. The user then moves the device to a next position at which the device again irradiates another portion of the skin with a pulse of treatment light. This process is repeated till the user has moved the device along the entire body part.

### SUMMARY OF THE INVENTION

The user is not able to see which part of the skin has already been irradiated and which part has not. As a result, many users struggle to hold the device at the correct positions to irradiate the entire body part in a uniform way. In case the user does not move the device over a sufficiently large distance between irradiations, parts of the skin are irradiated multiple times. Irradiating the skin multiple times may lead to discomfort, pain or even burns. In case the user moves the device over a too large distance between irradiations, parts of the skin are not irradiated. The hairs in these parts of the skin remain. This may result in poor depilation results.

It is an objective to provide an improved light-based skin treatment device that has a good depilation result and reduces discomfort.

According to a first aspect of the invention, there is provided a light-based skin treatment device comprising a treatment light source, a sensor light source, a light sensor and a controller. The treatment light source is adapted to provide treatment light to the skin. The sensor light source is adapted to irradiate a skin portion with sensor light. The light sensor is arranged to receive reflected sensor light from the skin portion. The light sensor is adapted to generate a sensor signal representative of a skin color based on the reflected sensor light. The controller is configured to receive from the light sensor a first sensor signal representative of a skin color of a first skin portion. The controller is configured to receive from the light sensor a second sensor signal representative of a skin color of a second skin portion. The controller is configured to determine first monochromatic data based on the first sensor signal. The controller is configured to determine second monochromatic data based on the second sensor signal. The controller is configured to determine a difference between the first monochromatic data and the second monochromatic data. The controller is configured to generate a treatment signal based on the difference. The treatment signal is indicative of the light-based skin treatment device being in a suitable position relative to the skin to provide treatment light to the skin.

The expression "skin color" is usually used as a single expression to represent the color of the entire skin of a person. For example, a person has a white skin color or a black skin color. However, skin color varies across the body of a person. This variation is, for example, caused by color variations of the skin, darker and lighter portions of the skin, variations in hair density, the presence or absence of moles or freckles, or visible veins under the skin. Also any matter on the skin, such as makeup residue or dirt, affects the perception of the skin color. The invention makes use of the insight that when comparing the skin color of the first skin portion with the skin color of the second skin portion, the difference indicates whether the first skin portion and the second skin portion overlap or are two different skin portions. In case of a large overlap, the difference is small. If the difference is small, the light-based skin treatment device is too close to an area of the skin that was previously irradiated with the treatment light. If the difference is large enough, the light-based skin treatment device faces a sufficiently large portion of the skin that was not irradiated with treatment light previously. If the difference is large enough, the treatment signal indicates that the light-based skin treatment device is in a suitable position relative to the skin to provide treatment light to the skin. This allows the user to operate the light-based skin treatment device to provide treatment light to the skin. This way, overtreatment of the skin is reduced or prevented. Further, the difference is determined between the first monochromic data and the second monochromic data. The first monochromic data and the second monochromic data represent the skin color only in a single color component. The inventors have discovered that the monochromic data is sufficient to detect changes in skin color while moving the light sensor across the skin. As only a single color component is used instead of multiple colors components, only a minimal amount of computing is required to determine the difference. This allows the difference to be calculated by the controller with an increased speed. As the controller is able to generate the treatment signal quickly, the time needed to treat the complete body part is reduced. As a result, the user is able to use the light-based skin treatment device to obtain good depilation results in a shorter time, while reducing discomfort and pain.

For example, the light-based skin treatment device is a photo-epilation device. For example, the light-based skin treatment device is an intense pulsed light (IPL) device. For example, the light-based skin treatment device is an intense pulsed light (IPL) device adapted to perform photo-epilation.

The treatment light source is, for example, adapted to provide light pulses. For example, the treatment light source is adapted to generate light at a high intensity for a short duration, such as for less than 100 ms or less than 50 ms or less than 10 ms or less than 8 ms. The intensity of the light pulse is high enough to perform an operation on the skin. Such operation is, for example, hair removal or photo-rejuvenation or vein treatment or acne treatment. For example, the treatment light source comprises one or more optical filters. For example, the treatment light source comprises an optical filter to prevent light with a potentially damaging wavelength, such as UV light, from being transmitted to the skin. For example, the treatment light source comprises a Xenon flash lamp, or a laser, or multiple lasers, or a LED, or multiple LEDs. For example, the treatment light source is adapted to provide treatment light with wavelengths in the range of 530-1200 nanometers. For example, the light-based skin treatment apparatus is an intense pulsed light (IPL) apparatus, wherein the treatment light source is an IPL light source.

The sensor light source is, for example, a Light-Emitting Diode, LED. For example, the sensor light source is adapted to generate as sensor light monochromatic radiation, such as red light or green light or blue light. For example, the sensor light source is adapted to generate as sensor light polychromatic radiation, such as white light.

The sensor light source is adapted to irradiate a skin portion with sensor light. For example, the sensor light is directly emitted from the sensor light source to the skin. In another example, the sensor light is emitted from the sensor light source via one or more optical components to the skin. For example, the one or more optical components comprise a window or a mirror or a lens or a diffusor.

When the sensor light is incident on the skin, the sensor light interacts with the skin. For example, part of the sensor light is reflected by the skin towards the light sensor, whereas another part of the sensor light is absorbed by the skin. For example, the sensor light reflected from the skin to the light sensor has less wavelengths or other wavelengths than the sensor light as emitted from sensor light source. For example, the sensor light reflected from the skin to the light sensor has less intensity in total or less intensity for one or more wavelengths than the sensor light as emitted from the sensor light source.

The light sensor is, for example, a photodetector. The light sensor comprises, for example, a photodiode or multiple photodiodes. For example, the light sensor comprises a phototransistor or multiple photo-transistors. For example, the light sensor comprises a charged-coupled device, CCD. For example, the light sensor comprises a CMOS image sensor. The light sensor is arranged to receive the reflected sensor light from the skin directly or via one or more optical components. For example, the one or more optical components comprise a window, or a mirror, or a lens. For example, the light sensor is arranged to receive all the reflected sensor light from the skin, or only a portion of the reflected sensor light form the skin.

The light sensor is able to generate the first sensor signal at a different time than the second sensor signal. For example, the light sensor generates first the first sensor signal, and some time thereafter the second sensor signal. The difference in time allows the light-based skin treatment device to be moved to a different position relative to the skin.

The controller is configured to receive the first sensor signal and the second sensor signal. For example, the controller is electrically or optically coupled to the light sensor to receive the first sensor signal and the second sensor signal. For example, the controller comprises a memory to store the first sensor signal while waiting for the second sensor signal to be received. For example, the controller comprises a memory to store the monochromatic data while waiting for the second sensor signal to be received. For example, the controller is configured to convert polychromatic data from the first sensor signal and the second sensor signal into the first monochromatic data and the second monochromatic data respectively. For example, the first monochromatic data and the second monochromatic data comprise information about a brightness or a luminosity. For example, the controller is configured to determine first monochromatic data and the second monochromatic data using 128 or 256 or 1024 or more brightness levels.

The controller is configured to determine the difference between the first monochromic data and the second monochromic data. For example, the difference is between an average brightness value of the skin color of the first skin portion and an average brightness value of the skin color of the second skin portion. For example, the difference is between a brightness profile along the first skin portion and a brightness profile along the second skin portion. For example, the difference is between a maximum brightness value of the first skin portion and a maximum brightness value of the skin color of the second skin portion. For example, the difference is between a minimum brightness value of the first skin portion and a minimum brightness value of the skin color of the second skin portion.

The controller generates the treatment signal based on the difference. In case the difference is large enough, the controller generates the treatment signal. For example, the difference is large enough if a difference or a ratio between the first monochromatic data and the second monochromatic data exceeds a threshold. For example, the difference is large enough if a difference or a ratio between the average brightness of the first skin portion and the average brightness of the second skin portion exceeds a threshold. For example, the difference is representative of an overlap of the first skin portion and the second skin portion of less than 10% or less than 5% or less than 1%. For example, the difference is representative of no overlap between the first skin portion and the second skin portion.

In an embodiment, the controller is configured to determine a displacement of the light-based skin treatment device relative to the skin based on the difference, and to generate the treatment signal based on the displacement.

According to this embodiment, the controller makes use of the insight that if the skin color of the first skin portion and the second skin portion are different from each other, the first skin portion and the second skin portion are at a distance from each other. For example, in case the difference between the first monochromatic data and the second monochromatic data indicates that there is no overlap between the first skin portion and the second skin portion, the controller is able to determine that the displacement of the light-based skin treatment device is at least a size of the first skin portion 221. For example, in case the difference between the first monochromatic data and the second monochromatic data indicates that there is an overlap between the first skin portion and the second skin portion, the controller is able to determine that the displacement of the light-based skin treatment device corresponds to the length of the overlap. For example, in case the overlap is 50% or 25%, then the displacement is 50% or 75% of the size of the first skin portion respectively. Based on the displacement, the controller is configured to generate the treatment signal. For example, when the displacement corresponds to a size of the treatment area irradiated by the treatment light while the device is at a certain position relative to the skin, the controller generates the treatment signal. This way, successive treatment areas are arranged adjacent to each other.

In an embodiment, the first monochromatic data comprises a first gray value. The second monochromatic data comprises a second gray value. The difference is between the first gray value and the second gray value.

According to this embodiment, the controller determines gray values to represent the skin color. The gray values provide sufficient information about the skin color of the first skin portion and the second skin portion, while only little computing power is needed to calculate the difference between the gray values. For example, the first sensor signal comprises a red color component, a green color component, and a blue color component. The first gray value is determined, for example, adding the intensity values of the red color component, the green color component, and the blue color component, and then to divide by three. The first gray value is determined, for example, by using a weighted method that weighs the color components according to the wavelengths of the color components. For example, the first gray value is determined by 0.299•R+0.587•G+0.114•B, wherein R is the intensity value of the red color component, G is the intensity value of the green color component, and B is the intensity value of the blue color component. For example, the first gray value is determined based on the a RGB to YUV conversion. For example, the second gray value is determined in the same way as the first gray value. For example, the skin color of the first skin portion is represented by a single gray value, and the skin color of the second skin portion is represented by a single gray value. For example, first monochromatic data comprises only the first gray value, and second monochromatic data comprises only the second gray value.

In an embodiment, the sensor light source is arranged to irradiate the first skin portion when the light-based skin treatment device is at a first position relative to the skin. The sensor light source is arranged to irradiate the second skin portion when the light-based skin treatment device is at a second position relative to the skin. The light sensor is arranged to receive reflected sensor light from the first skin portion when the light-based skin treatment device is at the first position relative to the skin. The light sensor is arranged to receive reflected sensor light from the second skin portion when the light-based skin treatment device is at the second position relative to the skin.

According to this embodiment, the light-based skin treatment device is at the first position relative to the skin. When the light-based skin treatment device is at the first position, the sensor light source irradiates the first skin portion 221, and the light sensor generates the first sensor signal based on the reflected sensor light from the first skin portion 221. Then, the light-based skin treatment device is at the second position relative to the skin. When the light-based skin treatment device is at the second position, the sensor light source irradiates the second skin portion, and the light sensor generates the second sensor signal based on the reflected sensor light from the second skin portion. This way, the difference between the first monochromatic data and the second monochromatic data is representative of a displacement of the light-based skin treatment device from the first position to the second position.

In an embodiment, the controller is configured to determine, based on the difference, whether a distance between the first position and the second position exceeds a threshold.

According to this embodiment, the controller determines whether the distance exceeds a threshold. When the distance exceeds a threshold, the first position and the second position are sufficiently far apart for the treatment light source to provide the treatment light to the skin. Because the first position and the second position are sufficiently far apart, there is no or almost no overtreatment of the skin. When the distance does not exceed the threshold, the first position and the second position are not sufficiently far apart for the treatment light source to provide the treatment light to the skin. As the first position and the second position are too close to each other, overtreatment would occur if treatment light were provided to the skin at the first position and at the second position.

In an embodiment, the treatment light source is adapted to provide treatment light to the skin in response to the treatment signal.

According to this embodiment, the treatment light source provides treatment light to the skin when receiving the treatment signal. This allows the user to move the light-based skin treatment device over the body part, while the controller determines when the treatment light source provides treatment light. The treatment light source does not provide treatment light in absence of the treatment signal. As a result, the user is able to quickly cover the body part with a reduced risk of overtreatment.

In an embodiment, the controller is configured to block the treatment light source from providing treatment light to the skin in absence of the treatment signal.

According to this embodiment, the absence of the treatment signal indicates that there is too much overlap between the first skin portion and the second skin portion. Providing treatment light when such a large overlap is present would cause overtreatment of the skin. As the controller blocks the treatment light from being provided, overtreatment is reduced or prevented. For example, the user is able to press a switch to cause the treatment light source to provide treatment light. When the controller blocks the treatment light from being provided, pressing the switch would not cause the treatment light source to provide treatment light.

In an embodiment, the light-based skin treatment device comprises a signal generator adapted to provide a perceivable signal for the user in response to the treatment signal. The perceivable signal is indicative to the user to operate the light-based skin treatment device to provide the treatment light to the skin.

According to this embodiment, the signal generator is a device that is able to generate the perceivable signal for the user, such as a visual signal or an auditory signal or a tactile signal. For example, the signal generator comprises a light or a display to provide a visual signal. For example, the signal generator comprises an indicator light that generates a green light in response to the treatment signal. For example, the indicator light does not generate the green light or generates a red light in absence of the treatment signal. For example, the signal generator comprises a sound generator or a speaker to generate an auditory signal, such as a beep, or a tune or a voice message. For example, the signal generator comprises a vibrator or a shaker to generate a tactile signal. As the user holds the light-based skin treatment device during use, the user is able to feel the tactile signal. When the user perceives the perceivable signal, the user is made aware by the signal generator that the device is in a suitable position to provide treatment light. The user is directly prompted to operate the device to irradiate the skin with the treatment light. For example, the device has a switch that the user can press to operate the device to expose the skin in response to the perceivable signal.

In an embodiment, the treatment light source is adapted to provide the treatment light in pulses. The controller is configured to limit a number of pulses in response to the difference being less than a threshold.

According to this embodiment, the device is provided with a safety feature that prevents any part of the skin from receiving more than a safe number of pulses of treatment light. For example, the safe number is 2, meaning that any portion of the skin may receive not more than 2 pulses of treatment light. As long as the difference is less than the threshold, there is too much overlap between the first skin portion and the second skin portion. The skin at the overlap receives a maximum of 2 pulses of treatment light, preventing overtreatment. If the device is not moved any further, the controller stops the treatment light source from providing any more treatment light. Only if the device is moved further relative to the skin, and the difference between the first skin portion and the second skin portion exceeds the threshold, the controller allows the treatment light to provide treatment light again. For example, the safe number is 1 or 3.

In an embodiment, the light-based skin treatment device comprises a treatment window adapted to expose a treatment area of the skin to the treatment light. The treatment window is adapted to expose the treatment area having the same size as the first skin portion.

According to this embodiment, the size of the treatment area is the same as the size of the first skin portion. As a result, overlap between the first skin portion and the second skin portion directly relates to an overlap between the treatment area and another, adjacent treatment area. For example, when the difference indicates that there is no overlap between the first skin portion and the second skin portion, there is also no overlap between the treatment area and the adjacent treatment area. For example, when the difference indicates that there is an overlap between the first skin portion and the second skin portion, the same overlap is between the treatment area and the adjacent treatment area. This allows the treatment area to be determined more accurately.

In an embodiment, the sensor light source is arranged to irradiate the first skin portion when the light-based skin treatment device is at a first position relative to the skin. The treatment light source is arranged to expose a treatment area of the skin when the light-based skin treatment device is at the first position. The first skin portion and the treatment area at least partly overlap with each other.

According to this embodiment, the first skin portion is at least partly irradiated with the treatment light. By overlapping the first skin portion and the treatment area, it is determined with improved accuracy whether the device is in a proper position to provide treatment light to the skin.

In an embodiment, the light sensor is adapted to generate the sensor signal comprising multiple color signals. Each color signal is representative of a different color component of the skin color. The controller is adapted to determine the first monochromatic data and the second monochromatic data based on the multiple color signals.

According to this embodiment, the first monochromatic data and the second monochromatic data are, for example, based on only one color of the multiple color signals, or based on an average value of the multiple color signals, or on a maximum or minimum value of the multiple color signals.

In an embodiment, the light sensor comprises an RGB sensor. The multiple color signals comprise a red color signal, a green color signal, and a blue color signal.

According to this embodiment, the first monochromatic data and the second monochromatic data are based on an RGB-signal from the RGB sensor. Since the RGB sensor is sensitive to changes of the skin color in red, green and blue, the difference of the skin color between the first skin portion and the second skin portion can be determined accurately. For example, the first monochromatic data comprises a first gray value. The first gray value is determined, for example, adding the intensity values of the red color signal, the green color signal, and the blue color signal, and then to divide by three. The first gray value is determined, for example, by using a weighted method that weighs the color signal according to the wavelengths of the color components. For example, the first gray value is determined by 0.299•R+0.587•G+0.114•B, wherein R is the intensity value of the red color component, G is the intensity value of the green color component, and B is the intensity value of the blue color component. For example, the first gray value is determined based on the a RGB to YUV conversion. For example, the second gray value is determined in the same way as the first gray value. For example, the skin color of the first skin portion is represented by a single gray value, and the skin color of the second skin portion is represented by a single gray value. For example, first monochromatic data comprises only the first gray value, and second monochromatic data comprises only the second gray value.

In an embodiment, the light sensor is an image sensor.

In a second aspect of the invention, there is provided a computer-implemented method. The method comprises receiving a first sensor signal representative of a skin color of a first skin portion. The method comprises receiving a second sensor signal representative of a skin color of a second skin portion. The method comprises determining first monochromatic data based on the first sensor signal. The method comprises determining second monochromatic data based on the second sensor signal. The method comprises determining a difference between the first monochromic data and the second monochromic data. The method comprises generating a treatment signal based on the difference. The treatment signal is indicative of a light-based skin treatment device being in a suitable position relative to a skin to provide treatment light to the skin.

In an embodiment, the computer-implemented method comprises determining a displacement of the light-based skin treatment device relative to the skin based on the difference, and generating the treatment signal based on the displacement.

In an embodiment, the first monochromatic data comprises a first gray value. The second monochromatic data comprises a second gray value. The difference is between the first gray value and the second gray value.

In an embodiment, each of the first sensor signal and the second sensor signal comprise multiple color signals. Each color signal is representative of a different color component of the skin color. The method comprises determining the first monochromatic data and the second monochromatic data based on the multiple color signals.

In an embodiment, the multiple color signals comprise a red color signal, a green color signal, and a blue color signal.

In a third aspect of the invention, there is provided a computer program product comprising instructions, which when executed by a controller of a light-based treatment device of the first aspect, causes the method according to the second aspect to be carried out.

### BRIEF DESCRIPTION OF THE DRAWINGS

Exemplary embodiments will now be described, by way of example only, with reference to the following Figures, in which:
FIG. 1 depicts a light-based skin treatment apparatus according to a first embodiment of the invention;
FIGs. 2-4 depict the light-based skin treatment device according to the first embodiment in various positions relative to the skin;
FIG. 5 depicts the controller for the light-based treatment device according to the first embodiment;
FIG. 6 depicts part of the light-based treatment device according to a second embodiment;
FIG. 7 depicts an operation of the light-based treatment device according to a third embodiment;
FIG. 8 depicts part of the light-based treatment device according to a fourth embodiment;
FIG. 9 depicts a computer-implemented method according to a fifth embodiment.

### DETAILED DESCRIPTION OF EMBODIMENTS

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

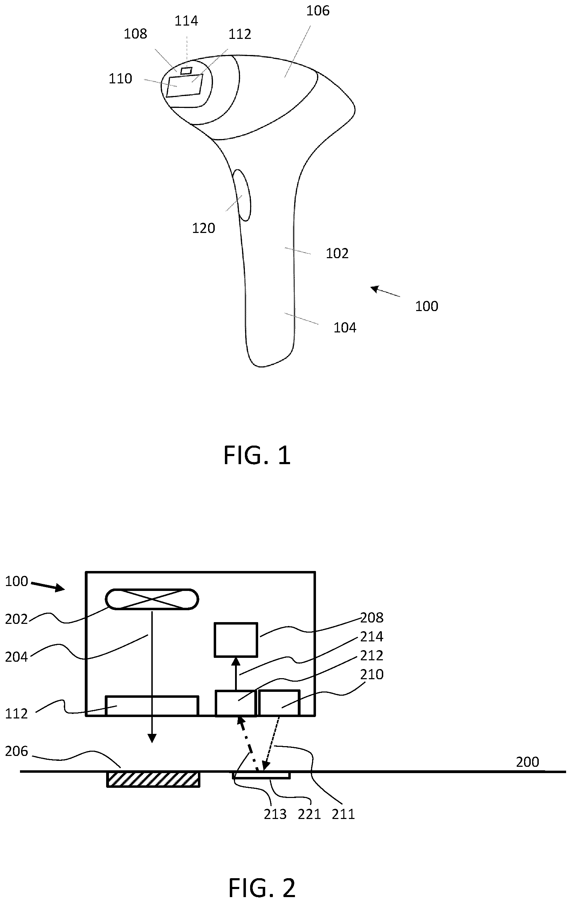

FIG. 1 depicts a light-based skin treatment device 100, such as an Intense Pulsed Light device, according to a first embodiment of the invention. The light-based skin treatment device 100 is adapted for use on the skin of a subject, e.g., a person or an animal. The light-based skin treatment device 100 comprises a housing 102 that has a handle portion 104 and a head portion 106. The handle portion 104 is shaped to enable the user to hold the light-based skin treatment device 100. The head portion 106 has a head end 108 that is to be placed into contact with the subject in order for the light-based skin treatment device 100to perform a treatment operation on the skin of the subject.

The light-based skin treatment device 100 comprises a treatment light source adapted to provide treatment light to the skin. The treatment light source is arranged inside the head portion 106. The Light-based skin treatment device 100 performs the treatment operation by providing pulses of the treatment light to the skin via the aperture 110. The aperture 110 is arranged in or on the housing 102 so that the aperture 110 can be placed adjacent to or on (i.e. in contact with) the skin of the subject. A treatment window 112 is arranged in the aperture 110. The intensity of the light pulses from the treatment light source are high enough to perform the treatment operation on the skin or body part adjacent to the aperture 110.

The light-based skin treatment device 100 comprises a user control 120 adapted to be operated by the user to activate the light-based skin treatment device 100. When the user activates the light-based skin treatment device 100 via the user control 120, the light-based skin treatment device 100 is able to perform the treatment operation. The user control 120 may be in the form of a switch, a button, a touch pad, etc.

FIGs 2-4 depict the light-based skin treatment device 100 according to the first embodiment in various positions relative to the skin.

FIG. 2 depicts the light-based skin treatment device 100. The light-based skin treatment device 100 comprises a treatment light source 202, a sensor light source 210, a light sensor 212, and a controller 208. The treatment light source 202 is adapted to provide treatment light 204 to the skin. The sensor light source 210 adapted to irradiate a portion of the skin 200 with sensor light 211. The light sensor 212 arranged to receive reflected sensor light 213 from the skin portion. The light sensor 212 is adapted to generate a sensor signal 214 representative of a skin color based on the reflected sensor light 213. In FIG. 2, the light-based skin treatment device 100is at a first position relative to the skin 200.

The controller 208 is configured to receive from the light sensor 212, a first sensor signal 214 representative of a skin color of a first skin portion 221.

When the light-based skin treatment device 100 is at the first position, the treatment window 112 exposes a part of the skin which is further referred to as the first treatment area 206.

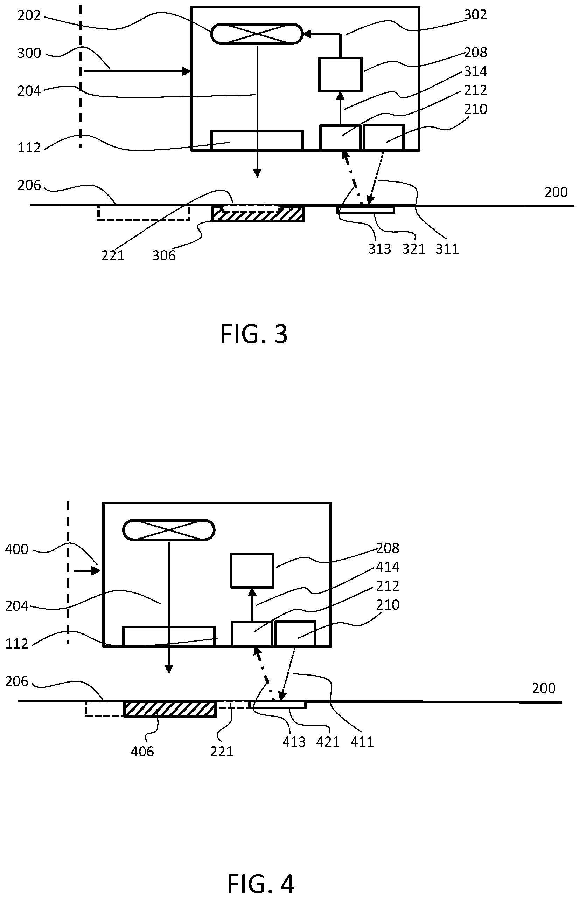

FIG. 3 depicts the light-based skin treatment device 100 at a second position relative to the skin 200. After the first treatment area 206 has been exposed to the treatment light, the user moves the light-based skin treatment device 100 along the skin 200 over a distance 300 till the light-based skin treatment device 100 reaches the second position. When in the second position, the sensor light source 210 irradiates a second skin portion 321 with sensor light 211. The light sensor 212 arranged to receive reflected sensor light 213 from the second skin portion 321. The light sensor 212 is adapted to generate a second sensor signal 314 representative of a skin color based on the reflected sensor light 313.

The controller 208 is configured to receive from the light sensor 212 the second sensor signal 314 representative of a skin color of a second skin portion 321. The controller 208 is configured to determine first monochromatic data based on the first sensor signal 214. The controller 208 is configured to determine second monochromatic data based on the second sensor signal 314. The controller 208 is configured to determine a difference between the first monochromatic data and the second monochromatic data. The controller 208 is configured to generate a treatment signal 302 based on the difference. The treatment signal 302 is indicative of the light-based skin treatment device 100 being in a suitable position relative to the skin to provide treatment light to the skin.

FIG. 4 depicts the light-based skin treatment device 100 at a position in between the first position and the second position. After the first treatment area 206 has been exposed to the treatment light 204, the user moves the light-based skin treatment device 100 along the skin 200 over a distance 400, which positions the light-based skin treatment device 100 at an intermediate position in between the first position and the second position. When in the intermediate position, the sensor light source 210 irradiates a third skin portion 421 with sensor light 411. The light sensor 212 arranged to receive reflected sensor light 413 from the third skin portion 421. The light sensor 212 is adapted to generate a third sensor signal 414 representative of a skin color based on the reflected sensor light 413.

The controller 208 is configured to receive from the light sensor 212 the third sensor signal 414 representative of a skin color of the third skin portion 421. The controller 208 is configured to determine first monochromatic data based on the first sensor signal 214. The controller 208 is configured to determine third monochromatic data based on the third sensor signal 414. The controller 208 is configured to determine a difference between the first monochromatic data and the third monochromatic data. The controller 208 determines that the difference between the first monochromatic data and the third monochromatic data is too small. The small difference represents that if the treatment light source 202 would provide treatment light 204 to the skin while the light-based treatment device 100 is in the intermediate position, the treatment light 204 would expose a third treatment area 406 that overlaps substantially with the first treatment area 206. Therefore, the controller 208 is configured to not generate the treatment signal 302 if the difference is too small. At the intermediate position, the light-based skin treatment device 100 is not in a suitable position relative to the skin to provide treatment light to the skin.

For example, the controller 208 is configured to determine a displacement of the light-based skin treatment device 100 relative to the skin 200 based on the difference, and to generate the treatment signal 302 based on the displacement. If the controller 208 determines the displacement is at least distance 300, there is no overlap between the first treatment area 206 and the second treatment area 306. In this case, the controller 208 generates the treatment signal 302. If the controller 208 determines the displacement is less than the distance 300, for example, the distance is only the distance 400, there would be overlap between the first treatment area 206 and the second treatment area 306. In this case, the controller 208 does not generate the treatment signal 302. For example, the controller 208 is configured to determine, based on the difference, whether a distance between the first position and the second position exceeds a threshold.

As depicted in FIGs. 2-4, the sensor light source 210 is arranged to irradiate the first skin portion 221 when the light-based skin treatment device 100 is at a first position relative to the skin 200. The sensor light source 210 is arranged to irradiate the second skin portion 321 when the light-based skin treatment device 100 is at the second position relative to the skin 200. The light sensor 212 is arranged to receive reflected sensor light 213 from the first skin portion 221 when the light-based skin treatment device 100 is at the first position relative to the skin. The light sensor 212 is arranged to receive reflected sensor light 313 from the second skin portion 321 when the light-based skin treatment device 100 is at the second position relative to the skin.

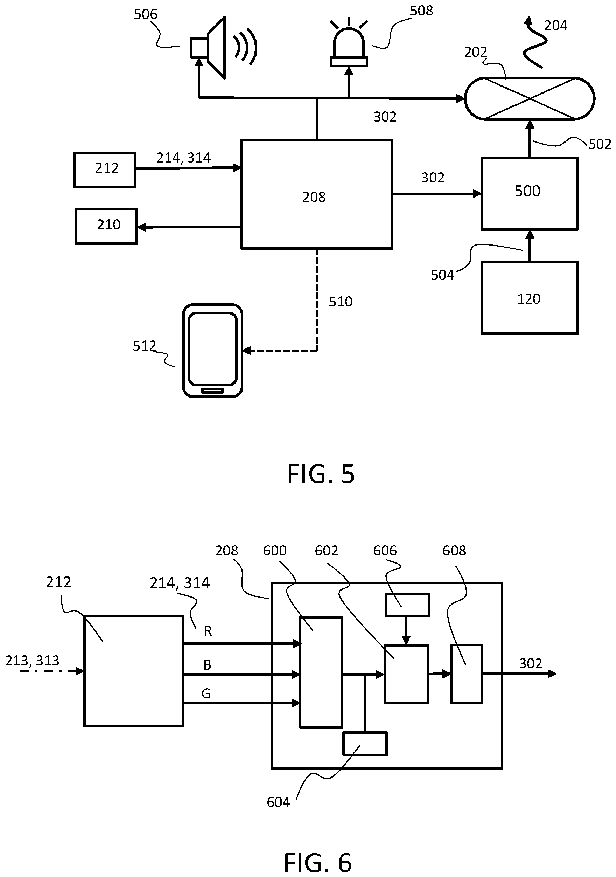

FIG. 5 depicts the controller 208 for the light-based treatment device 100 according to the first embodiment. The controller 208 is configured to control the sensor light source 210 to provide sensor light 211, 311, 411 to the skin. The controller 208 is configured to receive the sensor signal 214, 314, 414 from the light sensor 212. The controller 208 determines first monochromatic data based on the first sensor signal 214. The controller 208 determines the second monochromatic data based on the second sensor signal 314. The controller 208 determines the difference between the first monochromatic data and the second monochromatic data. If the controller 208 determines the difference is sufficiently large, the controller 208 generates the treatment signal 302. If the controller 208 determines the difference is too small, the controller 208 does not generate the treatment signal 302.

For example, the light-based skin treatment device 100 comprises a light source controller 500 adapted to control the treatment light source 202 to generate treatment light 204 in response to the treatment signal 302. The controller 208 provides the treatment signal 302 to the light source controller 500. The light source controller 500 provides a control signal 502 to the treatment light source 202. The control signal 502 causes the treatment light source 202 to generate treatment light 204.

For example, the light source controller 500 is adapted to block the treatment light source 202 from generating treatment light 204 in absence of the treatment signal 302. When the user operates the user control 120, the user control 120 provides a user signal 504 to the light source controller 500. The light source controller 500 is configured to generate the control signal 502 for the treatment light source 202 only in case the light source controller 500 receives the user signal 504 and the treatment signal 302. In absence of the treatment signal 302, the light source controller 500 does not provide the control signal 502 to the treatment light source 202, despite receiving the user signal 504.

For example, the light-based treatment device comprises an acoustic generator 506 adapted to generate an acoustic signal in response to the treatment signal 302. The acoustic generator 506 is, for example, a loudspeaker. When the controller 208 generates the treatment signal 302, the acoustic generator 506 provides an acoustic signal, such as a beep or sound or voice message. The acoustic signal prompts the user to operate the user control 120. The light source controller 500 receives the user signal 504 from the user control 120 and provides the control signal 502 to the treatment light source 202. The treatment light source 202 provides treatment light in response to the control signal 502. Optionally, the controller 208 provides the treatment signal 302 to both the acoustic generator 506 and the light source controller 500. The light source controller 500 is configured to generate the control signal 502 for the treatment light source 202 only in case the light source controller 500 receives the user signal 504 and the treatment signal 302. In absence of the treatment signal 302, the light source controller 500 does not provide the control signal 502 to the treatment light source 202.

For example, the light-based treatment device 100 comprises a visual signal generator 508 adapted to generate a visual signal in response to the treatment signal 302. For example, the visual signal generator 508 comprises an LED or an array of LEDs. When the controller 208 generates the treatment signal 302, the visual signal generator 508 provides a visual signal, such as a green light, or a blinking light, or a visual pattern. The visual signal prompts the user to operate the user control 120. The light source controller 500 receives the user signal 504 from the user control 120 and provides the control signal 502 to the treatment light source 202. The treatment light source 202 provides treatment light 204 in response to the control signal 502. Optionally, the controller 208 provides the treatment signal 302 to both the visual signal generator 508 and the light source controller 500. The light source controller 500 is configured to generate the control signal 502 for the treatment light source 202 only in case the controller 208 receives the user signal 504 and the treatment signal 302. In absence of the treatment signal 302, the light source controller 500 does not provide the control signal 502 to the treatment light source 202.

For example, the controller 208 is adapted to generate a display signal 510. The controller 208 is adapted to provide the display signal 510 to a display or a device having display, such as a mobile phone 512. When the controller 208 generates the treatment signal 302, the controller 208 generates the display signal 510. The display signal 510 causes the display to show an image or text on the display to the user to operate the user control 120. The light source controller 500 receives the user signal 504 from the user control 120 and provides the control signal 502 to the treatment light source 202. The treatment light source 202 provides treatment light 204 in response to the control signal 502. Optionally, the controller 208 provides the treatment signal 302 to both the visual signal generator 508 and the light source controller 500. The light source controller 500 is configured to generate the control signal 502 for the treatment light source 202 only in case the light source controller 500 receives the user signal 504 and the treatment signal 302. In absence of the treatment signal 302, the light source controller 500 does not provide the control signal 502 to the treatment light source 202.

As shown in FIG. 5, in an embodiment, the treatment light source 202 is adapted to provide treatment light 204 to the skin 200 in response to the treatment signal 302.

As shown in FIG. 5, in an embodiment, the controller 208 is configured to block the treatment light source 202 from providing treatment light 204 to the skin in absence of the treatment signal 302.

As shown in FIG. 5, in an embodiment, the light-based skin treatment device 100 comprises a signal generator, such as the acoustic generator 506, or the visual signal generator 508. The signal generator is adapted to provide a perceivable signal for the user in response to the treatment signal 302. The perceivable signal is indicative to the user to operate the light-based skin treatment device 100 to provide the treatment light to the skin.

FIG. 6 depicts part of the light-based treatment device 100 according to a second embodiment. The second embodiment has, for example, the same features as the first embodiment, except for the following.

In the second embodiment, the light sensor 212 is adapted to generate the sensor signal 214 comprising multiple color signals. The light sensor 212 is adapted to generate the second sensor signal 314 comprising multiple color signals The light sensor 212 comprises an RGB sensor. The RGB sensor is adapted to generate multiple color signals based on the received reflected light 213, 313. The multiple color signals are a red color signal (R), a green color signal (G), and a blue color signal (B). Each color signal is representative of a different color component of the skin color. The controller 208 is adapted to determine the first monochromatic data and the second monochromatic data based on the multiple color signals.

The controller 208 comprises a color converter 600, a comparator 602, a memory 604, a threshold memory 606, and a treatment signal 302 generator 608.

The color converter 600 is adapted to receive the multiple color signals. The color converter 600 is adapted to convert the multiple color signals into a monochromatic data. The color converter 600 generates the first monochromatic data based on the multiple color signals generated when the light-based treatment device 100 is at the first position. The color converter 600 generates the second monochromatic data based on the multiple color signals generated when the light-based treatment device 100 is at the second position. The color converter 600 provides the first monochromatic data and the second monochromatic data to the comparator 602. Optionally, the memory 604 stores the first monochromatic data and/or the second monochromatic data before providing the first monochromatic data and the second monochromatic data to the comparator 602. The comparator 602 compares the difference between the first monochromatic data and the second monochromatic data with a threshold form the threshold memory 606. In case the difference exceeds the threshold, the treatment signal generator 608 generates the treatment signal 302. In case the difference does not exceed the threshold, the treatment signal generator 608 does not generate the treatment signal 302.

For example, the light sensor 212 is an image sensor. For example, one or more of the color converter 600, the comparator 602, the memory 604, the threshold memory 606, and the treatment signal generator 608 are implemented in a single component, or have components in common with each other. For example, the controller 208 comprises a microprocessor configured to perform at least part of the function of the color converter 600, the comparator 602, and/or the treatment signal generator 608.

In an embodiment, the first monochromatic data comprises a first gray value. The second monochromatic data comprises a second gray value. The difference is between the first gray value and the second gray value. For example, monochromatic data is based on sensor signals from an RGB sensor. In case the first gray value is 1791, and the second gray value is 1765, the difference is large enough to represent two different portions of the skin 200, so without overlap between the two different portions of the skin 200. In case the first gray value is 1766 and the second gray value is 1767, the difference is small and represents overlapping portions of the skin 200. For example, the controller 208 determines there is no overlap if the difference between the first gray value and the second gray value is at least 20.

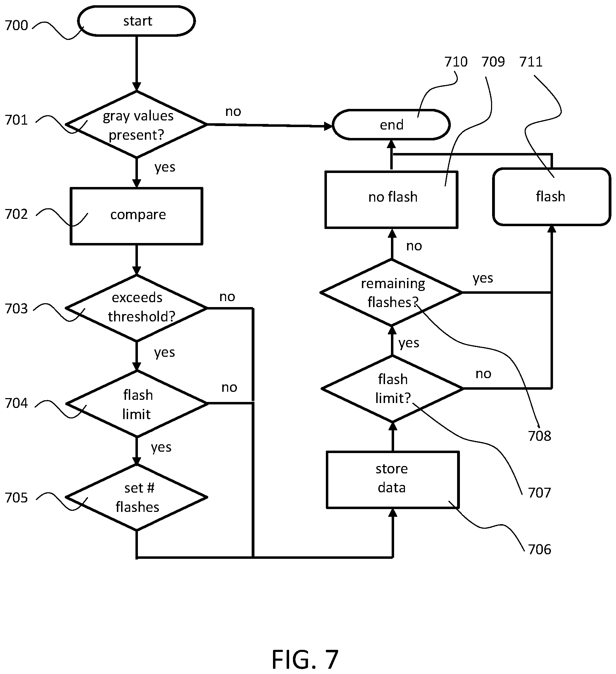

FIG. 7 depicts an operation of the light-based treatment device 100 according to a third embodiment. The third embodiment has, for example, the same features as the first embodiment, or the second embodiment, except for the following.

The controller 208 is configured to operate according to the flow diagram depicted in FIG. 7. At step 700, the operation of the controller 208 starts. At step 701, the controller 208 determines whether the first sensor signal 214 and the second sensor signal 314 have been received. The controller 208 determines the first monochromatic data and the second monochromatic data. The controller 208 determines whether the first monochromatic data and the second monochromatic data are present. For example, the first monochromatic data comprises the first gray value, and the second monochromatic data comprises the second gray value. In case the monochromatic data is present, the controller 208 continues to step 702. In step 702, the controller 208 determines the difference between the first monochromatic data and the second monochromatic data. Then, at step 703, the controller 208 determines whether the difference exceeds a threshold. In case the difference does not exceed the threshold, there is a large overlap between the first skin portion 221 and the second skin portion 321. Then, the controller 208 stores relevant data at step 706.

In case the difference exceeds the threshold, the controller 208 determines whether flash limiting is enabled. Flash limiting is a safety feature that limits the number of flashes for an area of the skin 200. A flash is a pulse of treatment light 204 that causes an operation on the skin. The flash limiting allows the light-based treatment device 100 to provide no more than a certain number of flashes per portion of the skin. Unless the light-based treatment device 100 is moved to another portion of the skin, the flash limiting blocks the treatment light source 202 to provide more than the certain number of flashes. If flash limiting is enabled, at step 705 the maximum number of flashes per area of skin is set. For example, the maximum number is 1 or 2 or 3. Then the relevant data is stored at step 706. The relevant data comprises the second monochromatic data, which the controller 208 can use for a next cycle through the flow diagram. The relevant data comprises the allowed maximum number of flashes. In case flash limiting is not enabled, step 705 is skipped, and the controller 208 stores the relevant data at step 706. In that case, the relevant data does not comprise the allowed maximum number of flashes.

After storing the data at step 706, the controller 208 has the required information for starting to provide treatment light 204 to the skin. At step 707, the controller 208 checks whether flash limiting is enabled. If no flash limiting is enabled, the controller 208 generates the treatment signal 302 in case the difference exceeds the threshold. The treatment signal 302 prompts the user or causes the light source controller 500 to provide a flash of treatment light 204 to the skin 200. The flash is indicated in step 711.

In case flash limiting is enabled, the controller 208 checks at step 708 how many flashes may still be provided to the current portion of the skin 200. After every flash, the controller 208 reduces the number of flashes to be provided with one. If the maximum number of flashes has not been reached yet, the controller 208 generates the treatment signal 302. If the maximum number of flashes has been reached, the controller 208 does not generate the treatment signal 302. In that case, no flash is provided as indicated instep 709. This way a portion of the skin receives only the maximum number of flashes. After either step 709 or step 711, the operation ends at step 710. Then the controller 208 restarts the operation at step 700. In the next cycle through the flow diagram, the number of flashes may be reset at step 705.

As indicated with the flash limit, the controller 208 is configured to limit the number of pulses in response to the difference being less than a threshold.

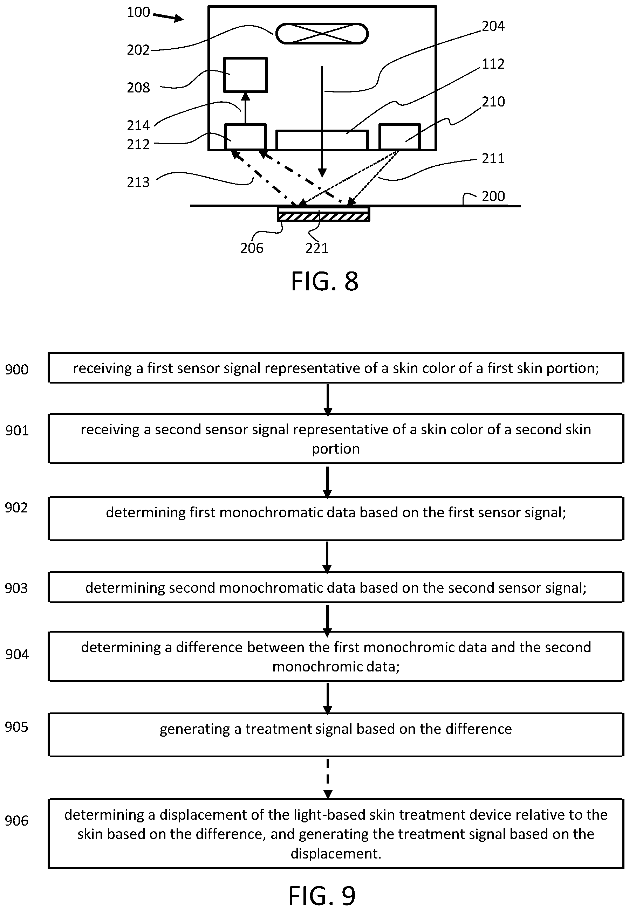

FIG. 8 depicts part of the light-based treatment device 100 according to a fourth embodiment. The fourth embodiment has, for example, the same features as the first embodiment, the second embodiment, and the third embodiment, except for the following.

The fourth embodiment comprises the treatment window 112 adapted to expose the first treatment area 206 of the skin 200 to the treatment light 204. The treatment window 112 is adapted to expose the first treatment area 206 having the same size as the first skin portion 221.

The sensor light source 210 is arranged to irradiate the first skin portion 221 when the light-based skin treatment device 100 is at the first position relative to the skin 200. The treatment light source 202 is arranged to expose the first treatment area 206 of the skin 200 when the light-based skin treatment device 100 is at the first position. The first skin portion 221 and the first treatment area 206 at least partly overlap with each other.

FIG. 9 depicts a computer-implemented method according to a fifth embodiment. The computer-implemented method according to the fifth embodiment is, for example, executed by the controller 208 according to any one of the first embodiment, the second embodiment, the third embodiment, or the fourth embodiment.

The computer-implemented method comprises, at 900, receiving the first sensor signal 214 representative of a skin color of a first skin portion 221. The computer-implemented method comprises, at 901, receiving the second sensor signal 214 representative of a skin color of the second skin portion 321. The computer-implemented method comprises, at 902, determining first monochromatic data based on the first sensor signal 214. The computer-implemented method comprises, at 903, determining second monochromatic data based on the second sensor signal 214. The computer-implemented method comprises, at 904, determining the difference between the first monochromic data and the second monochromic data. The computer-implemented method comprises, at 905, generating the treatment signal 302 based on the difference. The treatment signal 302 is indicative of a light-based skin treatment device 100 being in a suitable position relative to the skin 200 to provide treatment light 204 to the skin 200.

Optionally, the computer-implemented method comprises, at 906, determining a displacement of the light-based skin treatment device 100 relative to the skin 200 based on the difference, and generating the treatment signal 302 based on the displacement.

Optionally, the first monochromatic data comprises a first gray value. The second monochromatic data comprises a second gray value. The difference is between the first gray value and the second gray value.

Optionally, each of the first sensor signal 214 and the second sensor signal 214 comprise multiple color signals. Each color signal is representative of a different color component of the skin color. The computer-implemented method comprises determining the first monochromatic data and the second monochromatic data based on the multiple color signals. For example, the multiple color signals comprise a red color signal, a green color signal, and a blue color signal.

In a further embodiment, there is provided a computer program product comprising instructions, which when executed by the controller 208 of a light-based treatment device 100 of any one of the embodiments, causes the computer-implemented method according to the fifth embodiment to be carried out.

The light-based treatment device 100 according to any one of the embodiments described above, is, for example, used by a user at home. In another example, the light-based treatment device is used in a commercial environment, such as a beauty salon or a spa, by a professional user on a client. In that example, the professional may operate the light-based treatment device to perform photo-epilation on the skin of the client.

For example, the light-based treatment device is used in a non-therapeutic method, or in a cosmetic method, or in a non-therapeutic, cosmetic method. For example, the light-based treatment device is used in a non-therapeutic method for removing hairs.

As discussed above, the controller 208 is adapted to perform the data processing. The controller 208 can be implemented in numerous ways, with software and/or hardware, to perform the various functions required. The controller 208 may employ one or more microprocessors that may be programmed using software (e.g. microcode) to perform the required functions. The controller 208 may be implemented as a combination of dedicated hardware to perform some functions and one or more programmed microprocessors and associated circuitry to perform other functions.

The controller 208 may include circuitry. Examples of circuitry that may be employed include, but are not limited to, conventional microprocessors, application specific integrated circuits (ASICs), and field-programmable gate arrays (FPGAs). Thus, the controller 208 may be embodied as a digital and/or analog processing system.

In various implementations, the controller 208 may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or may be transportable, such that the one or more programs stored thereon can be loaded into a processing system.

Functions implemented by a processor may be implemented by a single processor or by multiple separate processing units which may together be considered to constitute a "processor". Such processing units may in some cases be remote from each other and communicate with each other in a wired or wireless manner.

A computer program may be stored/distributed on a suitable medium, such as an optical storage medium or a solid-state medium supplied together with or as part of other hardware, but may also be distributed in other forms, such as via the Internet or other wired or wireless telecommunication systems. The controller 208 may be configured to execute the computer program, causing the light-based treatment device 100 to perform the computer-implemented method according to the fifth embodiment.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A light-based skin treatment device (100), comprising:
a treatment light source (202) adapted to provide treatment light (204) to the skin (200);
a sensor light source (210) adapted to irradiate a skin portion with sensor light (211);
a light sensor (212) arranged to receive reflected sensor light (213) from the skin portion,
wherein the light sensor (212) is adapted to generate a sensor signal (214) representative of a skin color based on the reflected sensor light (213); and
a controller (208) configured to:
receive from the light sensor (212) a first sensor signal (214) representative of a skin color of a first skin portion (221);
receive from the light sensor (212) a second sensor signal (214) representative of a skin color of a second skin portion (321);
determine first monochromatic data based on the first sensor signal (214);
determine second monochromatic data based on the second sensor signal (214);
determine a difference between the first monochromatic data and the second monochromatic data;
generate a treatment signal (302) based on the difference,
wherein the treatment signal (302) is indicative of the light-based skin treatment device (100) being in a suitable position relative to the skin to provide treatment light to the skin.

2. The light-based skin treatment device (100) according to claim 1,
wherein the controller (208) is configured to determine a displacement of the light-based skin treatment device (100) relative to the skin based on the difference, and to generate the treatment signal (302) based on the displacement.

3. The light-based skin treatment device (100) according to claim 1 or 2,
wherein the first monochromatic data comprises a first gray value,
wherein the second monochromatic data comprises a second gray value,
wherein the difference is between the first gray value and the second gray value.

4. The light-based skin treatment device (100) according to any one of the preceding claims,
wherein the sensor light source (210) is arranged to irradiate the first skin portion (221) when the light-based skin treatment device (100) is at a first position relative to the skin,
wherein the sensor light source (210) is arranged to irradiate the second skin portion (321) when the light-based skin treatment device (100) is at a second position relative to the skin,
wherein the light sensor (212) is arranged to receive reflected sensor light (213) from the first skin portion (221) when the light-based skin treatment device (100) is at the first position relative to the skin,
wherein the light sensor (212) is arranged to receive reflected sensor light (213) from the second skin portion (321) when the light-based skin treatment device (100) is at the second position relative to the skin.

5. The light-based skin treatment device (100) according to any one of the preceding claims,
wherein the treatment light source (202) is adapted to provide treatment light to the skin in response to the treatment signal (302).

6. The light-based skin treatment device (100) according to any one of the preceding claims,
wherein the controller (208) is configured to block the treatment light source (202) from providing treatment light to the skin in absence of the treatment signal (302).

7. The light-based skin treatment device (100) according to any one of the preceding claims, comprising a signal generator (506, 508) adapted to provide a perceivable signal for the user in response to the treatment signal (302),
wherein the perceivable signal is indicative to the user to operate the light-based skin treatment device (100) to provide the treatment light to the skin.

8. The light-based skin treatment device (100) according to any one of the preceding claims,
wherein the treatment light source (202) is adapted to provide the treatment light in pulses, wherein the controller (208) is configured to limit a number of pulses in response to the difference being less than a threshold.

9. The light-based skin treatment device (100) according to any one of the preceding claims,
wherein the sensor light source (210) is arranged to irradiate the first skin portion (221) when the light-based skin treatment device (100) is at a first position relative to the skin,
wherein the treatment light source (202) is arranged to expose a treatment area of the skin when the light-based skin treatment device (100) is at the first position,
wherein the first skin portion (221) and the treatment area at least partly overlap with each other.

10. The light-based skin treatment device (100) according to any one of the preceding claims,
wherein the light sensor (212) is adapted to generate the sensor signal (214) comprising multiple color signals,
wherein each color signal is representative of a different color component of the skin color,
wherein the controller (208) is adapted to determine the first monochromatic data and the second monochromatic data based on the multiple color signals.

11. The light-based skin treatment device (100) according to claim 10,
wherein the light sensor (212) comprises an RGB sensor,
wherein the multiple color signals comprise a red color signal, a green color signal, and a blue color signal.

12. A computer-implemented method, comprising:
receiving a first sensor signal (214) representative of a skin color of a first skin portion (221);
receiving a second sensor signal (214) representative of a skin color of a second skin portion (321); determining first monochromatic data based on the first sensor signal (214);
determining second monochromatic data based on the second sensor signal (214);
determining a difference between the first monochromic data and the second monochromic data;
generating a treatment signal (302) based on the difference,
wherein the treatment signal (302) is indicative of a light-based skin treatment device (100) being in a suitable position relative to a skin to provide treatment light to the skin.

13. The computer-implemented method according to claim 12,
wherein the first monochromatic data comprises a first gray value,
wherein the second monochromatic data comprises a second gray value,
wherein the difference is between the first gray value and the second gray value.

14. The computer-implemented method according to any one of claims 12-13,
wherein each of the first sensor signal (214) and the second sensor signal (214) comprise multiple color signals,
wherein each color signal is representative of a different color component of the skin color,
wherein the method comprises
determining the first monochromatic data and the second monochromatic data based on the multiple color signals.

15. A computer program product comprising instructions, which when executed by a controller (208) of a light-based treatment device of any one of claims 1-11, causes the method according to claims 12-14 to be carried out.
